# EUROPÄISCHE PATENTSCHRIFT

(11) **EP 0 749 958 B1**
(45) Veröffentlichungstag und Bekanntmachung des Hinweises auf die Patenterteilung: **20.10.1999**
(21) Anmeldenummer: 96109316.8
(22) Anmeldetag: 11.06.1996
(51) Int. Cl.: C07C 263/10, C07C 265/10, C07C 265/14

(54) **Verfahren zur Herstellung von Triisocyanaten**
Process for the preparation of triisocyanates
Procédé de préparation de triisocyanates

(30) Priorität: 23.06.1995 DE 19523385
(43) Veröffentlichungstag der Anmeldung: 27.12.1996
(73) Patentinhaber: BAYER AG, 51368 Leverkusen (DE)
(72) Erfinder: Bischof, Eric, Dr., 42799 Leichlingen (DE); Dahmer, Jürgen, Dr., 51061 Köln (DE); Flink, Andreas, 41539 Dormagen (DE); Krohn, Wolfgang, Dr., 51061 Köln (DE); Molnar, Attila, Dr., 51519 Odenthal (DE)

(56) Entgegenhaltungen:
- EP-A- 0 289 840
- EP-A- 0 424 836
- EP-A- 0 570 799
- EP-A- 0 676 392
- DE-C- 19 510 259

## Beschreibung

Die Erfindung betrifft ein Verfahren zur Herstellung von nicht in technisch relevanter Ausbeute verfügbaren (cyclo)aliphatischen Triisocyanaten durch Phosgenierung der entsprechenden Triamine in der Gasphase.

Obwohl die Herstellung von organischen Isocyanaten durch Umsetzung von Aminen mit Phosgen in der Gasphase seit langem bekannt ist (z.B. Siefken, Justus Liebigs Anm. Chem. 562, 108 (1949)), wurde es bislang nur zur Herstellung von Monoisocyanaten (z.B. Ullmann, 4. Auflage, Band 13, Seite 353), von (cyclo)aliphatischen Diisocyanaten (EP-A-0 289 840 und die ältere eigene deutsche Patentanmeldung P 44 12 327.2) oder zur Herstellung der großtechnischen aromatischen Diisocyanate (DE-OS 4 217 019) empfohlen.

Obwohl (cyclo)aliphatische Triisocyanate in der Literatur Erwähnung finden, muß darauf verwiesen werden, daß diese Triisocyanate keine in technischen Mengen zur Verfügung stehenden Verbindungen darstellen. Diese Triisocyanate sind nämlich durch die klassische Phosgenierung der entsprechenden Triamine in der Flüssigphase nicht in technisch vertretbaren Ausbeuten zugänglich. Ursache hierfür sind neben den niedrigen Rohwareausbeuten mechanische Probleme bei der Herstellung nach diesem Verfahren.

So liefert die herkömmliche Phosgenierung von 1,8-Diamino-4-aminomethyloctan in der Flüssigphase Ausbeuten von 74 %, bezogen auf eingesetztes Amin (DE-C-31 09 276). Nachteilig sind neben der niedrigen Ausbeute mechanische Probleme, die ein zuverlässiges Rühren des Reaktionsgemisches erschweren bzw. extrem hohe Verdünnungen mit Lösungsmitteln während der Phosgenierung erforderlich machen. Die genannten Nachteile können gemäß der Lehre der JP-A-60 233 043 oder JP-A-60 233 044 durch Mischphosgenierung der Triamine mit aromatischen bzw. aliphatischen Diaminen teilweise verringert werden. Nachteilig bei diesen Verfahren ist jedoch die Notwendigkeit einer destillativen Trennung der beiden gebildeten Polyisocyanate sowie der Zwangsanfall des jeweiligen Diisocyanats.

Wie jetzt überraschenderweise gefunden wurde, gelingt es, die genannten Triisocyanate durch Phosgenierung der entsprechenden Triamine in ganz wesentlich erhöhten Ausbeuten unter Eliminierung der genannten Nachteile des Standes der Technik herzustellen, wenn die Phosgenierung der Triamine und die anschließende Aufarbeitung des Reaktionsgemischs in Analogie zur Verfahrensweise der EP-A-0 289 840 erfolgt. Eine derartige ganz wesentliche Ausbeuteverbesserung konnte nicht erwartet werden, da die schlechten Ausbeuten der Verfahren des Standes der Technik insbesondere auf Nebenreaktionen während der Phosgenierungsreaktion die durch die hohen Funktionalitäten der beteiligten Spezies bedingt sind, zurückzuführen ist, von denen nicht vorhersehbar war, daß sie bei der Gasphasenphosgenierung weitgehend unterbleiben.

Gegenstand der Erfindung ist ein Verfahren zur Herstellung von Triisocyanaten der allgemeinen Formel 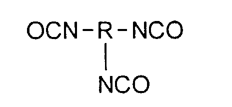 in welcher
- R: für einen (cyclo)aliphatischen Kohlenwasserstoffrest mit bis zu 22 Kohlenstoffatomen steht,
durch Phosgenierung der entsprechenden Triamine der allgemeinen Formel 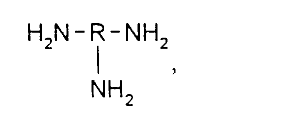 dadurch gekennzeichnet, daß man
a) die in der Dampfform vorliegenden, gegebenenfalls mit einem Inertgas oder mit den Dämpfen eines inerten Lösungsmittels verdünnten, auf eine Temperatur von 200 bis 600°C erhitzten Triamine einerseits und auf 200 bis 600°C erhitztes Phosgen andererseits kontinuierlich in einem auf 200 bis 600°C erhitzten, zylindrischen Reaktionsraum ohne sich bewegende Teile unter Aufrechterhaltung einer Strömungsgeschwindigkeit im Reaktionsraum von mindestens 3 m/s miteinander zur Reaktion bringt,
b) das den Reaktionsraum kontinuierlich verlassende Gasgemisch mit Hilfe eines inerten, flüssigen Lösungsmittels, welches bei einer Temperatur oberhalb der Zersetzungstemperatur des dem Triamin entsprechenden Carbamidsäurechlorid gehalten wird unter Gewinnung einer Lösung des Triisocyanats in diesem Lösungsmittel abkühlt und
c) das in dem inerten Lösungsmittel gelöst vorliegende Triisocyanat einer destillativen Aufarbeitung unterzieht.

Bei den bei dem erfindungsgemäßen Verfahren als Ausgangsmaterialien einzusetzenden Triaminen handelt es sich um solche der allgemeinen Formel 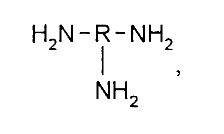 für welche R die bereits genannte Bedeutung hat. Vorzugsweise werden solche Triamine der genannten allgemeinen Formel eingesetzt, für welche
- R: für einen gesättigten (cyclo)aliphatischen Kohlenwasserstoffrest mit 4 bis 22, insbesondere 6 bis 15 und besonders bevorzugt 7 bis 11 Kohlenstoffatomen steht, wobei zwischen 2 Aminogruppen jeweils mindestens 2 Kohlenstoffatome angeordnet sind.

Die Bezeichnung "(cyclo)aliphatisch" soll bedeuten, daß in den Kohlenwasserstoffresten sowohl offenkettig aliphatische als auch cycloaliphatische Struktureinheiten vorliegen können, wobei die Aminogruppen sowohl aliphatisch als auch cycloaliphatisch gebunden sein können.

Zu den geeigneten bzw. bevorzugt geeigneten Triaminen gehören beispielsweise 1,8-Diamino-4-(aminomethyl)octan, 1,6,11-Undecantriamin, 1,7-Diamino-4-(3-aminopropyl)heptan, 1,6-Diamino-3-(aminomethyl)hexan oder 1,3,5-Tris(aminomethyl)-cyclohexan. Grundsätzlich können jedoch als Ausgangsmaterialien beliebige (cyclo)aliphatische Triamine mit bis zu 22 Kohlenstoffatomen eingesetzt werden, soweit sie unter den Temperaturbedingungen des erfindungsgemäßen Verfahrens stabil sind und in die Dampfform überführt werden können. Grundsätzlich als Ausgangsmaterial geeignet sind daher beliebige Triaminocyclohexane, Tris(aminomethyl)**-**cyclohexane, Triamino**-**methylcyclohexane oder vergleichbare Triamine.

Die Ausgangstriamine werden vor der Durchführung des erfindungsgemäßen Verfahrens verdampft und kontinuierlich auf eine Temperatur innerhalb des Temperaturbereichs von 200 bis 600°C, vorzugsweise 300 bis 500°C erhitzt. Die zuvor erhitzten Triamindämpfe können beim erfindungsgemäßen Verfahren als solche oder in mit Inertgas oder mit Dämpfen eines inerten Lösungsmittels verdünnter Form zum Einsatz gelangen. Die Durchmischung der Triamindämpfe mit dem Inertgas kann beispielsweise durch Verdampfen des Triamins im Strom eines inerten Gases oder der Dämpfe eines inerten Lösungsmittels erfolgen. Bevorzugtes Inertgas ist Stickstoff. Geeignete inerte Lösungsmittel, deren Dämpfe ebenfalls zur Verdünnung des Diamins verwendet werden können, sind beispielsweise Chlorbenzol, o-Dichlorbenzol, Xylol, Chlornaphthalin, Decahydronaphthalin oder deren Gemische.

Die Menge des gegebenenfalls als Verdünnungsmittel mitverwendeten Inertgases oder Lösungsmitteldampfes ist nicht kritisch, kann jedoch genutzt werden um die Verdampfungstemperatur des Amins abzusenken.

Das bei der Phosgenierung verwendete Phosgen wird, bezogen auf das Diamin, im Überschuß eingesetzt. Im allgemeinen genügt eine Phosgenmenge, die 150 bis 300 % der Theorie bezüglich der ablaufenden Phosgenierreaktion entspricht.

Vor der Durchführung des erfindungsgemäßen Verfahrens wird der Phosgenstrom auf eine Temperatur innerhalb des Bereichs von 200 bis 600°C, vorzugsweise 300 bis 500°C erhitzt.

Zur Durchführung der erfindungsgemäßen Umsetzung werden die vorerhitzten Ströme von Triamin bzw. Triamin-Inertgas**-**Gemisch einerseits und von Phosgen andererseits kontinuierlich in einen zylindrischen Reaktionsraum geleitet und dort miteinander vermischt.

Geeignete zylinderförmige Reaktionsräume sind beispielsweise Rohrreaktoren ohne Einbauten und ohne sich bewegende Teile im Innern des Reaktors. Die Rohrreaktoren bestehen im allgemeinen aus Stahl, Glas, legiertem oder emailliertem Stahl und weisen eine Länge auf, die ausreichend ist, um unter den Verfahrensbedingungen eine vollständige Umsetzung des Triamins mit dem Phosgen zu ermöglichen. Die Gasströme werden im allgemeinen an einem Ende des Rohrreaktors in diesen eingeleitet, wobei diese Einleitung beispielsweise durch an einem Ende des Rohrreaktors angebrachte Düsen oder durch eine Kombination aus Düse und einem Ringspalt zwischen Düse und Mischrohr erfolgen kann. Das Mischrohr wird ebenfalls bei einer Temperatur innerhalb des Bereichs von 200 bis 600°C, vorzugsweise 300 bis 500°C, gehalten, wobei diese Temperatur gegebenenfalls durch Beheizen des Reaktionsrohr aufrechterhalten wird.

Bei der Durchführung des erfindungsgemäßen Verfahrens liegt im allgemeinen der Druck in den Zuleitungen zum Reaktionsraum bei 200 bis 3000 mbar und am Ausgang aus dem Reaktionsraum bei 150 bis 2000 mbar, wobei durch Aufrechterhaltung eines geeigneten Differenzdrucks eine Strömungsgeschwindigkeit innerhalb des Reaktionsraums von mindestens 3, vorzugsweise mindestens 6 und besonders bevorzugt 10 bis 120 m/s Sorge getragen wird. Unter diesen Voraussetzungen herrschen innerhalb des Reaktionsraums im allgemeinen turbulente Strömungsverhältnisse vor.

Nach der erfolgten Phosgenierungsreaktion im Reaktionsraum wird das den Reaktionsraum kontinuierlich verlassende, gasförmige Gemisch von dem gebildeten Triisocyanat befreit. Dies kann beispielsweise mit Hilfe eines inerten Lösungsmittels erfolgen, dessen Temperatur so gewählt wird, daß sie einerseits oberhalb der Zersetzungstemperatur des dem Tiisocyanat entsprechenden Carbamidsäurechlorids und andererseits unterhalb der Kondensationstemperatur des Triisocyanats und vorzugsweise auch des gegebenenfalls in der Dampfform als Verdünnungsmittel mitverwendeten Lösungsmittels liegt, so daß Triisocyanat und Hilfslösungsmittel kondensieren bzw. sich in dem Lösungsmittel lösen, während überschüssiges Phosgen, Chlorwasserstoff und gegebenenfalls als Verdünnungsmittel mitverwendetes Inertgas die Kondensationsstufe bzw. das Lösungsmittel gasförmig durchlaufen. Zur selektiven Gewinnung des Triisocyanats aus dem den Reaktionsraum gasförmig verlassenden Gemisch besonders gut geeignet sind bei einer Temperatur von 120 bis 200°C, vorzugsweise 120 bis 170°C, gehaltene Lösungsmittel der oben beispielhaft genannten Art, insbesondere technisches Dichlorbenzol. Denkbare Methoden der selektiven Kondensation des gebildeten Diisocyanats aus dem den Reaktor verlassenden Gasgemisch unter Verwendung derartiger Lösungsmittel sind beispielsweise das Durchleiten des Gasgemisches durch das genannte Lösungsmittel oder das Eindüsen des Lösungsmittels (Lösungsmittelnebel) in den Gasstrom.

Das die Kondensationsstufe zur Gewinnung des Triisocyanats durchlaufende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol oder Dichlorbenzol) oder durch Adsorption und Hydrolyse an Aktivkühle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recyclisiert werden.

Die Reindarstellung der Triisocyanate erfolgt durch destillative Aufarbeitung der Lösung des Triisocyanats in dem zur Triisocyanatkondensation eingesetzten Lösungsmittel.

Im nachfolgenden Beispiel beziehen sich alle Prozentangaben auf Gewichtsprozente.

### Beispiel

In ein auf 510°C erwärmtes Mischrohr von 2,5 mm Durchmesser und 17,5 mm Länge mit nachgeschalteter Triisocyanatkondensationsstufe und dieser nachfolgenden, mit Aktivkohle gefülltem Phosgen-Adsorptionsturm, strömen kontinuierlich durch die Düse, die in das Mischrohr hineinragt, 8 Mol/Stunde Phosgen, daß in einem vorgeschalteten Wärmetauscher bei einem Druck von 950 mbar auf eine Temperatur von 460°C erwärmt wurde. Durch den Ringspalt zwischen Düse und Mischrohr wird gleichzeitig ein auf 410°C erwärmtes Gemisch aus 0,4 Mol/Stunde 4-Aminomethyl-1,8-octadiamin und 0,1 Mol/Stunde Stickstoff als Verdünnungsmittel in das Mischrohr geleitet. Die Strömungsgeschwindigkeit im Reaktionsraum liegt hierbei bei 100 m/s. Durch Anlegen eines Vakuums am Ende der Triisocyanatkondensationsstufe wird ein Druck im Mischrohr von ca. 350 mbar aufrechterhalten. Das heiße, den Reaktionsraum gasförmig verlassende Reaktionsgemisch wird in der Kondensationsstufe durch Dichlorbenzol geleitet, welches bei einer Temperatur von 150 bis 160°C gehalten wird. Hierbei erfolgt die selektive Kondensation des gebildeten Triisocyanats. Das die Waschstufe durchlaufende, im wesentlichen aus Stickstoff, Chlorwasserstoff und überschüssigem Phosgen bestehende Gemisch wird in dem Adsorptionsturm anschließend vom Phosgen befreit. Das Triisocyanat wird aus dem Waschlösungsmittel destillativ in reiner Form gewonnen. Die Ausbeute an reinem Triisocyanat liegt bei 92 % der Theorie. Das gleiche Triisocyanat wurde im Beispiel 2 von DE-C-31 09 276 lediglich in einer Ausbeute von 74 % der Theorie erhalten.

## Patentansprüche

1. Verfahren zur Herstellung von Triisocyanaten der allgemeinen Formel 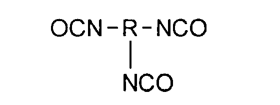 in welcher
R für einen (cyclo)aliphatischen Kohlenwasserstoffrest mit bis zu 22 Kohlenstoffatomen steht,
durch Phosgenierung der entsprechenden Triamine der allgemeinen Formel 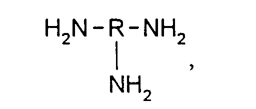 dadurch gekennzeichnet, daß man
a) die in der Dampfform vorliegenden, gegebenenfalls mit einem Inertgas oder mit den Dämpfen eines inerten Lösungsmittels verdünnten, auf eine Temperatur von 200 bis 600°C erhitzten Triamine einerseits und auf 200 bis 600°C erhitztes Phosgen andererseits kontinuierlich in einem auf 200 bis 600°C erhitzten, zylindrischen Reaktionsraum ohne sich bewegende Teile unter Aufrechterhaltung einer Strömungsgeschwindigkeit im Reaktionsraum von mindestens 3 m/s miteinander zur Reaktion bringt,
b) das den Reaktionsraum kontinuierlich verlassende Gasgemisch mit Hilfe eines inerten, flüssigen Lösungsmittels, welches bei einer Temperatur oberhalb der Zersetzungstemperatur des dem Triamin entsprechenden Carbamidsäurechlorid gehalten wird unter Gewinnung einer Lösung des Triisocyanats in diesem Lösungsmittel abkühlt und
c) das in dem inerten Lösungsmittel gelöst vorliegende Triisocyanat einer destillativen Aufarbeitung unterzieht.

2. Verfahren gemäß Anspruch 1, dadurch gekennzeichnet, daß man als zu phosgenierendes Triamin 1,8-Diamino-4-(aminomethyl)octan, 1,6,11-Undecantriamin, 1,7-Diamino-4-(3-aminopropyl)heptan, 1,6-Diamino-3-(aminomethyl)hexan oder 1,3,5-Tris(aminomethyl)-cyclohexan verwendet.

3. Verfahren gemäß Anspruch 1 und 2, dadurch gekennzeichnet, daß man die Umsetzung in dem Reaktionsraum bei einer Temperatur von 300 bis 500°C durchführt.

4. Verfahren gemäß Anspruch 1 bis 3, dadurch gekennzeichnet, daß man als Inertgas zur Verdünnung des dampfförmigen Triamins Stickstoff verwendet.

## Claims

1. A process for preparing triisocyanates of the general formula 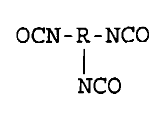 in which
R represents a (cyclo)aliphatic hydrocarbon group with up to 22 carbon atoms,
by phosgenation of the corresponding triamines of the general formula 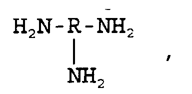 characterised in that
a) triamines which are present in the vapour form, optionally diluted with an inert gas or with the vapour of an inert solvent, are heated to a temperature of 200 to 600°C on the one hand, and phosgene heated to 200 to 600°C on the other hand, are continuously reacted together in a cylindrical reaction chamber heated to 200 to 600°C, without any moving parts, while maintaining a rate of flow in the reaction chamber of at least 3 m/s,
b) the gas mixture continuously leaving the reaction chamber is cooled with the assistance of an inert, liquid solvent which is held at a temperature above that of the decomposition temperature of the carbamic acid chloride corresponding to the triamine, with recovery of a solution of the triisocyanate in this solvent and
c) the triisocyanate which is dissolved in the inert solvent is subjected to working-up by distillation.

2. A process according to Claim 1, characterised in that 1,8-diamino-4-(aminomethyl)-octane, 1,6,11-undecanetriamine, 1,7-diamino-4-(3-aminopropyl)-heptane, 1,6-diamino-3-(aminomethyl)-hexane or 1,3,5-tris-(aminomethyl)-cyclohexane is used as the triamine to be phosgenated.

3. A process according to Claims 1 and 2, characterised in that the reaction is performed in the reaction chamber at a temperature of 300 to 500°C.

4. A process according to Claims 1 to 3, characterised in that nitrogen is used as the inert gas for diluting the triamine vapour.

## Revendications

1. Procédé pour la préparation de triisocyanates répondant à la formule générale 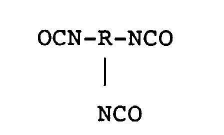 dans laquelle
R représente un radical d'hydrocarbure (cyclo)-aliphatique contenant jusqu'à 22 atomes de carbone,
par phosgénation des triamines correspondantes répondant à la formule générale 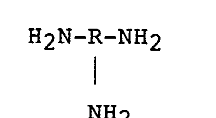 caractérisé en ce que
a) on amène à réagir l'un avec l'autre les triamines présentes à l'état de vapeur, le cas échéant diluées avec un gaz inerte ou avec les vapeurs d'un solvant inerte, chauffées à une température de 200 à 600°C, d'une part et du phosgène chauffé à une température de 200 à 600°C, d'autre part, en continu, dans un espace de réaction cylindrique chauffé à une température de 200 à 600°C, en l'absence d'éléments mobiles, tout en maintenant une vitesse d'écoulement dans l'espace de réaction d'au moins 3 m/s,
b) on refroidit le mélange gazeux quittant l'espace de réaction en continu, à l'aide d'un solvant liquide inerte qui est maintenu à une température supérieure à la température de décomposition du chlorure d'acide carbamique correspondant à la triamine, pour obtenir une solution du triisocyanate dans ce solvant, et
c) on soumet le triisocyanate présent à l'état dissous dans le solvant inerte à un traitement par distillation.

2. Procédé selon la revendication 1, caractérisé en ce qu'on utilise, à titre de triamine qui doit être soumise à une phosgénation, le 1,8-diamino-4-(aminométhyl)octane, la 1,6,11-undécanetriamine, le 1,7-diamino-4-(3-aminopropyl)heptane, le 1,6-diamino-3-(aminométhyl)hexane ou le 1,3,5-tris-(aminométhyl)cyclohexane.

3. Procédé selon les revendications 1 et 2, caractérisé en ce qu'on effectue la mise en réaction dans l'espace de réaction à une température de 300 à 500°C.

4. Procédé selon les revendications 1 à 3, caractérisé en ce qu'on utilise de l'azote à titre de gaz inerte à des fins de dilution de la triamine à l'état de vapeur.
